(19) **Europäisches Patentamt / European Patent Office / Office européen des brevets**

(11) **EP 3 833 447 B1**

(12) **FASCICULE DE BREVET EUROPEEN**

(45) Date de publication et mention de la délivrance du brevet:
**26.02.2025 Bulletin 2025/09**

(21) Numéro de dépôt: **19735606.6**

(22) Date de dépôt: **09.07.2019**

(51) Classification Internationale des Brevets (IPC):
**A61Q 19/00** (2006.01)    **A61K 8/34** (2006.01)
**A61K 8/92** (2006.01)    **A61K 8/37** (2006.01)

(52) Classification Coopérative des Brevets (CPC):
**A61Q 19/00; A61K 8/34; A61K 8/342; A61K 8/37; A61K 8/922;** A61K 2800/31

(86) Numéro de dépôt international:
**PCT/EP2019/068446**

(87) Numéro de publication internationale:
**WO 2020/030367 (13.02.2020 Gazette 2020/07)**

(54) **COMPOSITION COSMETIQUE ANHYDRE COMPRENANT DE L'HUILE VÉGÉTALE**

WASSERFREIE KOSMETISCHE ZUSAMMENSETZUNG ENTHALTEND PFLANZENÖLE

ANHYDROUS COSMETIC COMPOSITION COMPRISING VEGETABLE OIL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.08.2018 FR 1870907**

(43) Date de publication de la demande:
**16.06.2021 Bulletin 2021/24**

(73) Titulaire: **Laboratoires Clarins**
**75017 Paris (FR)**

(72) Inventeurs:
• **FOUCAULT, Sophie**
**78570 Andresy (FR)**
• **OPEL, Caroline**
**78500 Sartrouville (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**FR-A- 1 271 011       FR-A- 1 271 011**
**FR-A1- 3 061 009    JP-A- H05 186 328**

**Description**

DOMAINE TECHNIQUE

La présente invention concerne le domaine des compositions pour le soin des matières kératiniques comprenant des huiles végétales.

CONTEXTE

Depuis ces dernières années, les fabricants de cosmétiques font face à une demande croissante de la part des consommateurs pour des produits cosmétiques plus naturels et plus respectueux de la peau. Une multitude de compositions cosmétiques à base d'huiles végétales ont été mises sur le marché afin de répondre à cette demande. Les huiles végétales sont réputées pour leurs propriétés nourrissantes et sont particulièrement appréciées par les consommateurs en raison de leur naturalité et leur côté multifonction. En effet, ces huiles peuvent être utilisées aussi bien sur le visage que sur le corps et les cheveux. Il existe de nombreuses variétés d'huiles végétales ayant chacune des propriétés différentes. Cependant, un inconvénient majeur lié à l'utilisation de ces huiles est qu'elles peuvent parfois paraître trop grasses au toucher et pénétrer trop lentement lors de leur application sur la peau. De même, certaines de ces huiles peuvent avoir une odeur trop prononcée pour certains utilisateurs.

Afin de remédier à ces différents problèmes, la demanderesse a essayé d'ajouter de l'alcool aux huiles végétales afin d'obtenir une composition huileuse plus fluide et plus légère, au toucher sec, c'est-à-dire capable de pénétrer rapidement dans la peau en ne laissant pas de film gras, et ayant un impact olfactif limité. Cependant, les compositions huileuses obtenues après ajout d'alcool peuvent être troubles en raison de la non miscibilité des huiles végétales et de l'alcool, ce qui en plus d'être peu attrayant sur le plan visuel peut avoir pour conséquence que l'huile ne se répartisse pas de manière uniforme sur la peau ou les cheveux, lors de son application.

Par conséquent, il existe toujours un besoin pour des compositions huileuses limpides, fluides, légères et au toucher sec.

De plus, il existe un besoin pour des compositions huileuses simples avec un nombre d'ingrédients limité, un impact olfactif limité et respectueuses de l'environnement. FR 1 271 011 divulgue des méthodes de solubilisation des huiles dans des alcools de bas poids moléculaire et l'utilisation de ces compositions dans la domaine de la cosmétique.

La demanderesse a découvert de façon inattendue et surprenante que l'ensemble de ces problèmes peuvent être résolus par la composition selon la présente invention.

RÉSUMÉ

Selon un aspect, la présente invention a pour objet une composition cosmétique anhydre comprenant :

a) au moins une huile végétale en une teneur totale en poids **x** comprise entre 10% et 40% par rapport au poids total de la composition; et

b) au moins un monoalcool en C2-C4, en une teneur totale en poids **y** comprise entre 5% et 21% par rapport au poids total de la composition ; et

c) au moins un composé C différent des composés constituant l'huile végétale, en une teneur totale en poids **z** par rapport au poids total de la composition, le composé C ayant une valeur de logP calculée inférieure ou égale à 8,6, dans laquelle :

- le composé C est choisi dans le groupe constitué par les esters d'acide gras liquides, les esters d'alcool gras liquides, les alcools gras liquides et leurs mélanges ; et
- la composition ne comprend pas de charges ; et

$$90\,\% \leq \mathbf{x} + \mathbf{y} + \mathbf{z} \leq 100\,\% \; ;$$

et

$$\mathbf{z} \geq (0{,}21\mathbf{y} - 0{,}56)\,\mathbf{x}.$$

Selon un autre aspect, la présente invention concerne un procédé non thérapeutique de soin des matières kératiniques comprenant l'application sur les matières kératiniques d'une composition telle que définie ci-dessus.

DESCRIPTION DÉTAILLÉE DE CERTAINS MODES DE RÉALISATION PRÉFÉRÉS

Par "composition cosmétique anhydre" on entend une composition cosmétique comprenant une teneur totale en poids d'eau de moins de 0.5%, de préférence moins de 0.3 %, plus préférentiellement moins de 0.1% par rapport au poids total de la composition, le plus préférentiellement une composition cosmétique exempt d'eau.

Par "huile végétale" on entend un corps gras extrait d'une plante oléagineuse qui est liquide à température ambiante (25°C) et pression atmosphérique (1,013. $10^5$ Pa) et qui comprend de 95% à 99% en poids de triglycérides, par rapport au poids total du corps gras.

Par "monoalcool en C2-C4" on entend un alcool comprenant un seul groupe hydroxyle et une chaine carbonée linéaire ou ramifiée comprenant de 2 à 4 atomes de carbone.

Par "ester" on entend un monoester, un diester ou un triester issu de la réaction de monoacides ou diacides ou triacides carboxyliques, linéaires ou ramifiés ou cycliques, saturés ou insaturés avec des monoalcools ou des polyols, linéaires ou ramifiés ou cycliques, saturés ou insaturés.

Par "acide gras" on entend un acide carboxylique comportant de 4 à 22 atomes de carbone.

Par "ester d'acide gras liquide" on entend un ester d'acide gras qui est liquide à température ambiante (25°C) et pression atmosphérique (1,013. $10^5$ Pa).

Par "alcool gras" on entend un alcool non glycérolé et non oxyalkyléné, saturé ou insaturé, linéaire ou ramifié ou cyclique comportant de 8 à 30 atomes de carbone, de préférence de 10 à 30 atomes de carbone, plus préférentiellement de 12 à 25 atomes de carbone.

Par "oxyalkyléné" on entend un radical divalent -R-O- dans lequel R représente un radical alkylène linéaire en C1-C4 ou ramifié en C3-C4.

Par "alcool gras liquide" on entend un alcool gras qui est liquide à température ambiante (25°C) et pression atmosphérique (1,013. $10^5$ Pa).

Par "ester d'alcool gras liquide" on entend un ester d'alcool gras qui est liquide à température ambiante (25°C) et pression atmosphérique (1,013. $10^5$ Pa).

Par "charges" on entend des particules de toute forme, incolores ou blanches, minérales ou de synthèse, insolubles dans le milieu de la composition quelle que soit la température à laquelle la composition est fabriquée. Les charges peuvent être des charges organiques et/ou inorganiques.

Par "organique" on entend tout composé ou polymère dont la structure chimique comprend au moins un atome de carbone.

Par "inorganique" on entend tout composé ou polymère dont la structure chimique ne comprend pas d'atome de carbone.

Par "gélifiant lipophile" on entend un composé apte à gélifier une phase grasse.

Par "coefficient de partage n-octanol/eau" d'un composé on entend le rapport entre la concentration à l'équilibre d'un composé dans le n-octanol et la concentration en ce même composé dans l'eau.

Par "logP calculé" d'un composé on entend le logarithme décimal du coefficient de partage n-octanol/eau d'un composé calculé en utilisant le logiciel KOWWIN™ v1.68 inclus dans la suite de logiciels EPI (Estimation Programs Interface) Suite™ développée par US Environmental Protection Agency's Office of Pollution Prévention and Toxics et Syracuse Research Corporation (SRC). Cette suite de logiciels est téléchargeable gratuitement à l'adresse suivante : https://www.epa.gov/tsca-screening-tools/epi-suitetm-estimation-program-interface.

Par "matières kératiniques" on entend la peau, les lèvres, les cheveux, les cils, les sourcils, le cuir chevelu, les ongles, en particulier d'êtres humains.

Par "composé d'origine végétale", on entend un composé directement issu de végétaux ou obtenu par transformation de végétaux, de préférence par transformation biologique de végétaux ou obtenu à partir d'au moins un autre composé d'origine végétale. Par exemple, un ester d'origine végétale peut être obtenu à partir d'un alcool et d'un acide carboxylique, l'alcool et/ou l'acide carboxylique étant d'origine végétale.

L'expression "au moins un(e)" est équivalente à l'expression "un(e) ou plusieurs".

L'expression "comprenant" englobe l'expression "consistant en".

L'expression "compris(e) entre ... et ..." doit se comprendre bornes incluses.

## Composition cosmétique anhydre

La présente invention a pour objet une composition cosmétique anhydre telle que définie ci-avant.

Comme expliqué ci-avant, la demanderesse a ajouté de l'alcool aux huiles végétales afin d'obtenir une composition huileuse plus fluide et plus légère, au toucher sec, c'est-à-dire capable de pénétrer rapidement dans la peau en ne laissant pas de film gras. Cependant, les huiles végétales étant peu ou pas miscibles dans l'alcool, les compositions obtenues sont troubles, ce qui en plus d'être visuellement peu attrayant peut résulter en une application non uniforme de l'huile sur la peau ou les cheveux. La demanderesse a découvert de manière surprenante qu'afin d'obtenir une composition huileuse transparente, il est nécessaire d'ajouter le(s) composé(s) C à la composition en respectant des proportions particulières entre la teneur en huile(s) végétale(s), la teneur en monoalcool(s) en C2-C4 et la teneur en composé(s) C.

Une telle composition est particulièrement intéressante car très simple à préparer en raison du faible nombre d'ingrédients compris dans la composition.

La composition ne comprend pas de charges car les charges ne sont pas facilement dispersibles dans une composition huileuse et ne permettent pas d'obtenir une composition transparente.

De préférence, 95 % $\leq$ x + y + z $\leq$ 100 %. Plus préférentiellement, 97 % $\leq$ x + y + z $\leq$ 100 %.

De préférence, la composition peut ne pas comprendre de composés choisis dans le groupe constitué par les éther-oxydes, les gélifiants lipophiles et leurs mélanges. Les gélifiants lipophiles peuvent en effet apporter de l'épaisseur à la composition ce qui va à l'encontre de l'obtention d'une composition huileuse au toucher sec.

Par éther-oxyde, on entend un composé organique de formule R-O-R', dans laquelle R et R' sont des groupes alkyles. Comme exemple d'éther-oxyde, on peut citer le dicaprylyl ether.

De préférence, la composition peut ne pas comprendre d'esters d'acide gras liquides ou d'esters d'alcool gras liquides autres que le composé C ou d'esters d'acide gras liquides autres que ceux constituant l'huile végétale.

La valeur de logP calculée du composé C peut de préférence être comprise entre 4,0 et 8,6, plus préférentiellement entre 4,0 et 7,7.

Le composé C peut de préférence être d'origine végétale. L'ajout d'un composé C d'origine végétale permet d'obtenir une composition huileuse encore plus respectueuse de l'environnement.

Comme décrit ci-avant, le composé C est choisi dans le groupe constitué par les esters d'acide gras liquides, les esters d'alcool gras liquides, les alcools gras liquides et leurs mélanges.

### Esters d'acide gras liquide et esters d'alcool gras liquides

Avantageusement, le composé C peut être choisi dans le groupe constitué par les esters d'acide gras liquides, les esters d'alcool gras liquides et leurs mélanges.

Les esters d'acide gras liquides et/ou les esters d'alcool gras liquides peuvent de préférence être d'origine végétale.

Un ester d'acide gras liquide d'origine végétale peut par exemple être obtenu par estérification d'un acide gras et d'un alcool, l'acide gras et/ou l'alcool étant d'origine végétale.

Un ester d'alcool gras liquide d'origine végétale peut par exemple être obtenu par estérification d'un acide et d'un alcool gras, l'acide et/ou l'alcool gras étant d'origine végétale.

### Monoesters d'acide gras liquides et monoesters d'alcool gras liquides

Le composé C peut être choisi dans le groupe constitué par les monoesters d'acide gras liquides, les monoesters d'alcool gras liquides et leurs mélanges.

Les monoesters d'acide gras liquides peuvent répondre à la formule $R_1C(=O)OR_1'$, dans laquelle $R_1$ représente un radical alkyle linéaire ou ramifié comprenant de 3 à 15 atomes de carbone et $R_1'$ représente un radical alkyle linéaire ou ramifié comprenant de 3 à 10 atomes de carbone. $R_1$ peut de préférence représenter un radical alkyle linéaire ou ramifié comprenant de 3 à 9 atomes de carbone, plus préférentiellement de 5 à 9 atomes de carbone.

Le nombre total d'atomes de carbone compris dans les radicaux $R_1$ et $R_1'$ peut de préférence être compris entre 8 et 22, plus préférentiellement entre 9 et 18.

### Diesters et triesters

Le composé C peut également être choisi dans le groupe constitué par les diesters d'acide gras liquides, les diesters d'alcools gras liquides, les triesters d'acide gras liquides, les triesters d'alcool gras liquides et leurs mélanges.

Selon un mode de réalisation préféré, le composé C peut être choisi dans le groupe constitué par les composés de nom INCI suivant : Isononyl Isononanoate, Dibutyl Adipate, Propylheptyl Caprylate, Dicaprylyl Carbonate, Caprylyl Caprylate, Caprylyl Caprate, Isopropyl Palmitate, Diisopropyl Sebacate, Isodecyl Neopentanoate, Hexyl Laurate, Isopropyl Myristate, Isopropyl Palmitate, Ethylhexyl Caprylate, Ethylhexyl Caprate, Triheptanoin et leurs mélanges.

### Alcools gras liquides

Avantageusement, le composé C peut être choisi parmi les alcools gras liquides.

Les alcools gras liquides peuvent de préférence être d'origine végétale.

Les alcools gras liquides peuvent être linéaires ou ramifiées ou cycliques, saturés ou insaturés.

Le composé C peut être choisi parmi les alcools gras liquides linéaires insaturés, de préférence parmi les alcools gras liquides linéaires insaturés comprenant de 11 à 18 atomes de carbone.

Le composé C peut être choisi parmi les alcools gras liquides ramifiés, de préférence parmi les alcools gras liquides ramifiés comprenant de 12 à 24 atomes de carbone.

Le composé C peut de préférence être choisi parmi les alcools gras liquides sélectionnés dans le groupe constitué par l'alcool linoléique, l'alcool linolénique, l'alcool oléique, l'alcool isocétylique, l'alcool isostéarylique, l'alcool undécylénique, le 2-octyl-1-dodécanol, le 2-butyl-1-octanol, le 2-hexyl-1-décanol, le 2-octyl-1-dodécanol, le 2-décyl-1-tetradécanol et leurs mélanges. Le composé C peut plus préférentiellement être le 2-octyl-1-dodécanol.

La teneur totale en poids en composé(s) C z peut de préférence être comprise entre 55% et 80% par rapport au poids total de la composition.

## Huile(s) végétale(s)

Comme décrit ci-avant, la composition selon la présente invention comprend au moins une huile végétale. L'huile végétale peut être choisie parmi les huiles vierges, les huiles raffinées et leurs mélanges.

Par "huile vierge" on entend une huile végétale issue d'une seule et unique pression à froid, ne subissant aucun traitement chimique et ne contenant pas d'additifs.

Par "huile raffinée" on entend une huile végétale extraite à l'aide d'un solvant et qui subit un ou plusieurs traitements sélectionnés parmi les traitements de neutralisation, décoloration, désodorisation ou wintérisation.

Selon un mode de réalisation préféré, l'huile végétale a une constante diélectrique $\varepsilon$ à 25°C supérieure ou égale à 2,5.

L'huile végétale utilisable dans la composition selon la présente invention peut être choisie dans le groupe constitué par l'huile de cameline, l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de coton, l'huile de pépins de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyaux d'abricot, l'huile d'olive, l'huile d'onagre, l'huile de palme, l'huile de passiflore, l'huile de pépins de raisin, l'huile de rosier, l'huile de ricin, l'huile de seigle, l'huile de sésame, l'huile de son de riz, l'huile de soja, l'huile de tournesol, et leurs mélanges. L'huile végétale peut de préférence être choisie dans le groupe constitué par l'huile de noisette, l'huile de jojoba, l'huile de macadamia, l'huile de camélia, l'huile de coriandre, l'huile d'olive et leurs mélanges.

La teneur totale en poids en huile(s) végétale(s) x peut de préférence être comprise entre 10% et 35% par rapport au poids total de la composition.

## Monoalcool(s) en C2-C4

Le monoalcool en C2-C4 peut de préférence être choisi dans le groupe constitué par l'éthanol, le propanol, l'isopropanol, le butan-1-ol, l'isobutanol et leurs mélanges. Selon un mode de réalisation préféré, le monoalcool en C2-C4 est l'éthanol.

A titre d'exemple, la composition selon la présente invention peut comprendre de l'éthanol en tant que monoalcool en C2-C4 et au moins un composé C choisi dans le groupe constitué par les esters d'acide gras liquides, les esters d'alcool gras liquides et leurs mélanges.

À titre d'exemple, la composition selon la présente invention peut comprendre de l'éthanol en tant que monoalcool en C2-C4 et au moins un composé C choisi parmi les alcools gras liquides.

La teneur totale en poids en monoalcool(s) en C2-C4 y peut de préférence être comprise entre 10% et 21% par rapport au poids total de la composition.

## Autre(s) composé(s) D

La composition selon la présente invention peut comprendre en outre au moins un composé D différent de l'huile végétale, du monoalcool en C2-C4 et du composé C. La teneur totale en composé(s) D peut être comprise entre 0,2% et 10%, de préférence entre 0,2% et 5%, plus préférentiellement entre 0,2% et 3% en poids par rapport au poids total de la composition. On peut utiliser en tant que composé D au moins un adjuvant et/ou au moins un actif classiquement utilisé(s) en cosmétique. À titre d'exemple, on peut citer les actifs liposolubles comme la vitamine E, les colorants naturels liposolubles, les antioxydants, le menthol et ses dérivés.

## Procédé

La présente invention a également pour objet un procédé non thérapeutique de soin des matières kératiniques comprenant l'application sur les matières kératiniques d'une composition telle que définie ci-avant. La composition est appliquée de préférence sur la peau.

## Utilisation

Un mode de réalisation divulgué ne faisant pas partie de la présente invention concerne l'utilisation d'au moins un composé C tel que défini ci-avant pour faciliter la solubilisation d'une huile végétale dans un monoalcool en C2-C4, de préférence choisi dans le groupe constitué par l'éthanol, le propanol, l'isopropanol, le butan-1-ol, l'isobutanol et leurs mélanges, plus préférentiellement l'éthanol.

De préférence, dans cette utilisation, la teneur totale x, en % en poids, en huile végétale, la teneur totale y, en % en poids, en monoalcool C2-C4, et la teneur totale en poids z, en % en poids, en composé C sont telles que :

$$90\ \% \leq \mathbf{x} + \mathbf{y} + \mathbf{z} \leq 100\ \% \ ;$$

et

$$\mathbf{z} \geq (0{,}21\mathbf{y} - 0{,}56)\ \mathbf{x}.$$

EXEMPLES

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières, sauf mention contraire.

Les compositions selon les exemples 1 à 43 ont été préparées selon le mode opératoire décrit ci-après afin de montrer la supériorité des compositions selon la présente invention par rapport à des compositions de nature différente. Les valeurs de logP calculées pour chacun des composés C compris dans les compositions sont indiquées dans le tableau ci-après. Le nom INCI "Alcohol" correspond au nom chimique "éthanol".

[Tableau 1]

| Composés (Noms INCI) | Exemple 1 (Comparatif) | Exemple 2 (Comparatif) | Exemple 3 (Comparatif) | Exemple 4 (Comparatif) |
|---|---|---|---|---|
| Alcohol | 10,0 | 10,0 | 10,0 | 10,0 |
| Corylus Avellana (Hazelnut) Seed Oil | 40,0 | 40,0 | 38,5 | 37,5 |
| Isononyl Isononanoate (DUB ININ de Stéarinerie Dubois) | | 50,0 | 51,5 | 52,5 |
| Diisopropyl Sebacate (DUB DIS de Stéarinerie Dubois) | 50,0 | | | |
| Ratio Huile Végétale/Composé C | 10:12,5 | 10:12,5 | 10:13,4 | 10:14 |
| **logP Composé C calculé** | **5,2** | **7,4** | **7,4** | **7,4** |
| **Miscibilité à J+1** | +/- | +/- | +/- | +/- |
| (0,21 **y** - 0.56) **x** | 61,60 | 61,60 | 61,60 | 57,75 |

[Tableau 2]

| Composés (Noms INCI) | Exemple 5 (Invention) | Exemple 6 (Invention) | Exemple 7 (Invention) | Exemple 8 (Comparatif) |
|---|---|---|---|---|
| Alcohol | 10,0 | 10,0 | 10,0 | 10,0 |
| Corylus Avellana (Hazelnut) Seed Oil | 35,0 | 35,0 | 35,0 | 35,0 |
| Isononyl Isononanoate (DUB ININ de Stéarinerie Dubois) | | 55,0 | | |

EP 3 833 447 B1

(suite)

| Composés (Noms INCI) | Exemple 5 (Invention) | Exemple 6 (Invention) | Exemple 7 (Invention) | Exemple 8 (Comparatif) |
|---|---|---|---|---|
| Isopropyl Palmitate (BASF) | | | 55,0 | |
| Ethylhexyl Palmitate (Cegesoft® C24 de BASF) | | | | 55,0 |
| Hexyldecanol (Eutanol® G16 de BASF) | 55,0 | | | |
| Ratio Huile Végétale/Composé C | 10:15,7 | 10:15,7 | 10:15,7 | 10:15,7 |
| **logP Composé C calculé** | **6,7** | **7,4** | **8,2** | **10,6** |
| **Miscibilité à J+1** | + | + | + | - |
| (0,21 **y** - 0.56) **x** | 53,90 | 53,90 | 53,90 | 53,90 |

[Tableau 3]

| Composés (Noms INCI) | Exemple 9 (Invention) | Exemple 10 (Invention) | Exemple 11 (Invention) | Exemple 12 (Invention) |
|---|---|---|---|---|
| Alcohol | 10,0 | 10,0 | 10,0 | 10,0 |
| Corylus Avellana (Hazelnut) Seed Oil | 30,0 | 30,0 | 16,05 | 16,05 |
| Propylheptyl Caprylate (Cetiol®) SenSoft de BASF) | | | | 73,95 |
| Isononyl Isononanoate (DUB ININ de Stéarinerie Dubois) | | 60,0 | 73,95 | |
| Diisopropyl Sebacate (DUB DIS de Stéarinerie Dubois) | 60,0 | | | |
| Ratio Huile Végétale/Composé C | 10:20 | 10:20 | 10:46,1 | 10:46,1 |
| **logP Composé C calculé** | **5,2** | **7,4** | **7,4** | **7,7** |
| **Miscibilité à J+1** | + | + | + | + |
| (0,21 **y** - 0.56) **x** | 46,20 | 46,20 | 24,72 | 24,72 |

[Tableau 4]

| Composés (Noms INCI) | Exemple 13 (Invention) | Exemple 14 (Invention) | Exemple 15 (Invention) | Exemple 16 (Invention) |
|---|---|---|---|---|
| Alcohol | 10,0 | 10,0 | 10,0 | 10,0 |
| Corylus Avellana (Hazelnut) Seed Oil | 12,86 | 10,0 | 10,0 | |
| Macadamia Ternifolia Seed Oil | | | | 10,0 |
| Dicaprylyl Carbonate (Cetiol® CC de BASF) | | 80,0 | | |
| Isononyl Isononanoate (DUB ININ de Stéarinerie Dubois) | 77,14 | | | 80,0 |
| Dibutyl Adipate (Cetiol® B de BASF) | | | 80,0 | |
| Ratio Huile Végétale/Composé C | 10:60,0 | 10:80,0 | 10:80,0 | 10:80,0 |
| **logP Composé C calculé** | **7,4** | **7,1** | **4,3** | **7,4** |
| **Miscibilité à J+1** | + | + | + | + |

7

(suite)

| Composés (Noms INCI) | Exemple 13 (Invention) | Exemple 14 (Invention) | Exemple 15 (Invention) | Exemple 16 (Invention) |
|---|---|---|---|---|
| (0,21 **y** - 0.56) **x** | 19,80 | 15,40 | 15,40 | 15,40 |

[Tableau 5]

| Composés (Noms INCI) | Exemple 17 (Invention) | Exemple 18 (Invention) | Exemple 19 (Invention) | Exemple 20 (Invention) |
|---|---|---|---|---|
| Alcohol | 10,0 | 10,0 | 10,0 | 10,0 |
| Corylus Avellana (Hazelnut) Seed Oil | | 10,0 | 10,0 | 10,0 |
| Camellia Oleifera Seed Oil | 10,0 | | | |
| Propylheptyl Caprylate (Cetiol® SenSoft de BASF) | | | | 80,0 |
| Isononyl Isononanoate (DUB ININ de Stéarinerie Dubois) | 80,0 | | 80,0 | |
| Octyl Dodecanol (DUB ODOL de Stéarinerie Dubois) | | 80,0 | | |
| Ratio Huile Végétale/Composé C | 10:80,0 | 10:80,0 | 10:80,0 | 10:80,0 |
| **logP Composé C calculé** | **7,4** | **8,6** | **7,4** | **7,7** |
| **Miscibilité à J+1** | + | + | + | + |
| (0,21 **y** - 0.56) **x** | 15,40 | 15,40 | 15,40 | 15,40 |

[Tableau 6]

| Composés (Noms INCI) | Exemple 21 (Invention) | Exemple 22 (Invention) | Exemple 23 (Comparatif) | Exemple 24 (Comparatif) |
|---|---|---|---|---|
| Alcohol | 10,0 | 10,0 | 10,0 | 10,0 |
| Corylus Avellana (Hazelnut) Seed Oil | | 10,0 | 10,0 | 10,0 |
| Simmondsia Chinensis (Jojoba) Seed Oil | 10,0 | | | |
| Caprylyl Caprylate/Caprate (Cetiol® RLF de BASF) | 80,0 | 80,0 | | |
| Ethylhexyl Stéarate (DUB SO de Stéarinerie Dubois) | | | | 80,0 |
| Cetyl Ethylhexanoate (Tegosoft® CO de Evonik) | | | 80,0 | |
| Ratio Huile Végétale/Composé C | 10:80,0 | 10:80,0 | 10:80,0 | 10:80,0 |
| **logP Composé C calculé** | **6,8 à 7,7** | **6,8 à 7,7** | **10,6** | **11,6** |
| **Miscibilité à J+1** | + | + | - | - |
| (0,21 **y** - 0.56) **x** | 15,40 | 15,40 | 15,40 | 15,40 |

[Tableau 7]

| Composés (Noms INCI) | Exemple 25 (Comparatif) | Exemple 26 (Comparatif) | Exemple 27 (Comparatif) | Exemple 28 (Comparatif) |
|---|---|---|---|---|
| Alcohol | 10,0 | 10,0 | 13,55 | 13,5 |
| Corylus Avellana (Hazelnut) Seed Oil | 10,0 | 10,0 | 34,7 | 26,5 |
| Dicaprylyl Ether (Cetiol® OE de BASF) | 80,0 | | | |
| Isononyl Isononanoate (DUB ININ de Stéarinerie Dubois) | | | 51,75 | 60,0 |
| Isohexadecane | | 80,0 | | |
| Ratio Huile Végétale/Composé C | 10:80,0 | 10:80,0 | 10:14,9 | 10:22,6 |
| logP Composé C calculé | N/A Éther | N/A Alcane | 7,4 | 7,4 |
| Miscibilité à J+1 | - | - | - | +/- |
| (0,21 y - 0.56) x | | | 79,31 | 60,29 |

[Tableau 8]

| Composés (Noms INCI) | Exemple 29 (Invention) | Exemple 30 (Comparatif) | Exemple 31 (Invention) | Exemple 32 (Invention) |
|---|---|---|---|---|
| Alcohol | 13,5 | 15,0 | 15,0 | 15,0 |
| Corylus Avellana (Hazelnut) Seed Oil | 26,0 | 31,48 | 23,60 | 15,16 |
| Isononyl Isononanoate (DUB ININ de Stéarinerie Dubois) | 60,5 | 53,52 | 61,40 | 69,84 |
| Ratio Huile Végétale/Composé C | 10:23,3 | 10:17,0 | 10:26,0 | 10:46,1 |
| logP Composé C calculé | 7,4 | 7,4 | 7,4 | 7,4 |
| Miscibilité à J+1 | + | - | + | + |
| (0,21 y - 0.56) x | 59,15 | 81,53 | 61,12 | 39,26 |

[Tableau 9]

| Composés (Noms INCI) | Exemple 33 (Invention) | Exemple 34 (Comparatif) | Exemple 35 (Comparatif) | Exemple 36 (Comparatif) |
|---|---|---|---|---|
| Alcohol | 15,0 | 20,00 | 20,00 | 20,00 |
| Corylus Avellana (Hazelnut) Seed Oil | 14,16 | 32,00 | 29,00 | 26,50 |
| Isononyl Isononanoate (DUB ININ de Stéarinerie Dubois) | 70,84 | 48,0 | 51,00 | 53,50 |
| Ratio Huile Végétale/Composé C | 10:50 | 10:15,0 | 10:17,6 | 10:20,2 |
| logP Composé C calculé | 7,4 | 7,4 | 7,4 | 7,4 |
| Miscibilité à J+1 | + | - | - | - |
| (0,21 y - 0.56) x | 36,67 | 116,48 | 105,56 | 96,46 |

[Tableau 10]

| Composés (Noms INCI) | Exemple 37 (Invention) | Exemple 38 (Invention) | Exemple 39 (Invention) | Exemple 40 (Comparatif) |
|---|---|---|---|---|
| Alcohol | 20,20 | 20,20 | 20,20 | 20,0 |
| Corylus Avellana (Hazelnut) Seed Oil | 17,00 | 14,80 | 14,80 | 80,0 |
| Isononyl Isononanoate (DUB ININ de Stéarinerie Dubois) | 62,80 | | 65,00 | |
| Diisopropyl Sebacate (DUB DIS de Stéarinerie Dubois) | | 65,00 | | |
| Ratio Huile Végétale/Composé C | 10:36,9 | 10:43,9 | 10:43,9 | N/A Pas de composé C |
| logP Composé C calculé | **7,4** | **5,2** | **7,4** | **N/A Pas de composé C** |
| Miscibilité à J+1 | + | + | + | - |
| (0,21 **y** - 0.56) **x** | 62,59 | 54,49 | 54,49 | N/A Pas de composé C |

[Tableau 11]

| Composés (Noms INCI) | Exemple 41 (Comparatif) | Exemple 42 (Comparatif) | Exemple 43 (Comparatif) |
|---|---|---|---|
| Alcohol | 20,0 | 20,0 | 20,0 |
| Simmondsia Chinensis (Jojoba) Seed Oil | 80,0 | | |
| Macadamia Ternifolia Seed Oil | | 80,0 | |
| Olea Europaea (Olive) Fruit Oil | | | 80,0 |
| Ratio Huile Végétale/Composé C | N/A Pas de composé C | N/A Pas de composé C | N/A Pas de composé C |
| logP Composé C calculé | **N/A Pas de composé C** | **N/A Pas de composé C** | **N/A Pas de composé C** |
| Miscibilité à J+1 | - | - | - |
| (0,21 y - 0.56) x | N/A Pas de composé C | N/A Pas de composé C | N/A Pas de composé C |

Mode opératoire

Pour chacune des compositions, l'huile végétale est mélangée avec le composé C (si présent) dans un flacon sirop à l'aide d'un agitateur magnétique à une température de 20 ± 5°C. L'éthanol est ensuite ajouté dans le flacon puis le flacon est fermé. L'agitation est maintenue jusqu'à l'obtention d'un mélange limpide. Si au bout de 10 min, un mélange limpide n'est pas obtenu, l'agitation est arrêtée.

Observations

Dans le tableau ci-avant, le symbole (-) indique un mélange visuellement trouble obtenu lors de la préparation de la composition. Les compositions pour lesquelles un mélange limpide est obtenu sont vérifiées à J+1 (24h après la préparation de la composition) afin de s'assurer que les compositions sont toujours stables et qu'aucun trouble n'apparaît dans la composition. Le symbole (+) indique un mélange visuellement limpide obtenu lors de la préparation de la composition, celui-ci restant stable à J+1. Le symbole (+/-) indique un mélange visuellement limpide obtenu lors de la préparation de la composition mais non stable à J+1, un trouble commençant à se former au sein du mélange.

Conclusions

Il a été constaté que les compositions huileuses selon la présente invention (Exemples 5 à 7, 9 à 22, 29, 31 à 33, 37 à 39) sont limpides. De plus, ces compositions sont fraîches, fluides/légères et ont une odeur d'huile peu prononcée.

Il a également été constaté que les compositions huileuses ayant des caractéristiques différentes de celles selon la présente invention (Exemples 1 à 4, 8, 23 à 28, 30, 34 à 36, 40 à 43) sont visuellement troubles soit lors de leur préparation soit à J+1.

Les compositions des Exemples 1 à 4, 27, 28, 30 et 34 à 36 sont des compositions dont la teneur totale en poids en composé C est inférieure à celle de la présente invention.

Les compositions des exemples 40 à 43 comprennent uniquement de l'huile végétale et de l'alcool et ne comprennent pas de composé C tel que défini selon la présente invention. Les compositions des exemples 8, 23 et 24 comprennent des esters ayant un logP calculé qui est différent de celui des composés C tels que définis selon la présente invention. La composition de l'exemple 25 comprend un éther-oxyde qui n'est pas un composé C tel que défini selon la présente invention. La composition de l'exemple 26 comprend un alcane qui n'est pas un composé C tel que défini selon la présente invention.

Ainsi, lorsqu'une composition ne comprend pas de composé C tel que défini selon la présente invention ou dans une teneur différente de celle définie selon la présente invention un mélange visuellement trouble est obtenu soit immédiatement lors de la préparation de la composition soit à J+1. Un tel mélange peut avoir pour conséquence que l'huile peut ne pas se répartir de manière uniforme sur la peau ou les cheveux, lors l'application de la composition.

**Revendications**

1. Composition cosmétique anhydre comprenant:

a) au moins une huile végétale en une teneur totale en poids x comprise entre 10% et 40% par rapport au poids total de la composition; et
b) au moins un monoalcool en C2-C4, en une teneur totale en poids y comprise entre 5% et 21% par rapport au poids total de la composition ; et
c) au moins un composé C différent des composés constituant l'huile végétale, en une teneur totale en poids z par rapport au poids total de la composition, le composé C ayant une valeur de logP calculée inférieure ou égale à 8,6, de préférence entre 4,0 et 8,6, plus préférentiellement entre 4,0 et 7,7,

dans laquelle :

- le composé C est choisi dans le groupe constitué par les esters d'acide gras liquides, les esters d'alcool gras liquides, les alcools gras liquides et leurs mélanges ; et
- la composition ne comprend pas de charges ; et

$$90\ \% \le x + y + z \le 100\ \% \ ;$$

et

$$z \ge (0{,}21y - 0{,}56)\,x.$$

2. Composition selon la revendication 1, dans laquelle 95 % $\le$ x + y + z $\le$ 100 %, de préférence 97 % $\le$ x + y + z $\le$ 100 %.

3. Composition selon la revendication 1, dans laquelle la composition ne comprend pas de composés choisis dans le groupe constitué par les éther-oxydes, les gélifiants lipophiles et leurs mélanges.

4. Composition selon l'une quelconque des revendications précédentes, dans laquelle le monoalcool en C2-C4 est choisi dans le groupe constitué par l'éthanol, le propanol, l'isopropanol, le butan-1-ol, l'isobutanol et leurs mélanges, de préférence l'éthanol.

5. Composition selon l'une quelconque des revendications précédentes dans laquelle l'huile végétale a une constante diélectrique $\varepsilon$ à 25°C supérieure ou égale à 2,5.

**6.** Composition selon l'une quelconque des revendications précédentes, dans laquelle la composition ne comprend pas d'esters d'acide gras liquides ou d'esters d'alcool gras liquides autres que le composé C ou d'esters d'acide gras liquides autres que ceux constituant l'huile végétale.

**7.** Composition selon l'une quelconque des revendications précédentes, dans laquelle le composé C est d'origine végétale.

**8.** Composition selon l'une quelconque des revendications précédentes, dans laquelle le composé C est choisi dans le groupe constitué par les monoesters d'acide gras liquides, les monoesters d'alcool gras liquides et leurs mélanges.

**9.** Composition selon la revendication 8, dans laquelle les monoesters d'acide gras liquides répondent à la formule $R_1C(=O)OR_1'$, dans laquelle :

- $R_1$ représente un radical alkyle linéaire ou ramifié comprenant de 3 à 15 atomes de carbone, de préférence de 3 à 9 atomes de carbone, plus préférentiellement de 5 à 9 atomes de carbone ; et
- $R_1'$ représente un radical alkyle linéaire ou ramifié comprenant de 3 à 10 atomes de carbone.

**10.** Composition selon la revendication 9, dans laquelle le nombre total d'atomes de carbone compris dans les radicaux $R_1$ et $R_1'$ est compris entre 8 et 22, de préférence entre 9 et 18.

**11.** Composition selon l'une quelconque des revendications 1 à 7, dans laquelle le composé C est choisi dans le groupe constitué par les diesters d'acide gras liquides, les diesters d'alcools gras liquides, les triesters d'acide gras liquides, les triesters d'alcool gras liquides et leurs mélanges.

**12.** Composition selon l'une quelconque des revendications 1 à 7, dans laquelle le composé C est choisi dans le groupe constitué par les composés de nom INCI suivant : Isononyl Isononanoate, Dibutyl Adipate, Propylheptyl Caprylate, Dicaprylyl Carbonate, Caprylyl Caprylate, Caprylyl Caprate, Isopropyl Palmitate, Diisopropyl Sebacate, Isodecyl Neopentanoate, Hexyl Laurate, Isopropyl Myristate, Isopropyl Palmitate, Ethylhexyl Caprylate, Ethylhexyl Caprate, Triheptanoin et leurs mélanges.

**13.** Composition selon l'une quelconque des revendications 1 à 7, dans laquelle le composé C est choisi parmi les alcools gras liquides linéaires insaturés, de préférence parmi les alcools gras liquides linéaires insaturés comprenant de 11 à 18 atomes de carbone.

**14.** Composition selon l'une quelconque des revendications 1 à 7, dans laquelle le composé C est choisi parmi les alcools gras liquides ramifiés, de préférence parmi les alcools gras liquides ramifiés comprenant de 12 à 24 atomes de carbone.

**15.** Composition selon l'une quelconque des revendications 1 à 7, dans laquelle le composé C est choisi parmi les alcools gras liquides sélectionnés dans le groupe constitué par l'alcool linoléique, l'alcool linolénique, l'alcool oléique, l'alcool isocétylique, l'alcool isostéarylique, l'alcool undécylénique, le 2-octyl-1-dodécanol, le 2-butyl-1-octanol, le 2-hexyl-1-décanol, le 2-octyl-1-dodécanol et leurs mélanges, de préférence le 2-octyl-1-dodécanol.

**16.** Composition selon l'une quelconque des revendications précédentes, dans laquelle l'huile végétale est choisie dans le groupe constitué par l'huile de cameline, l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de coton, l'huile de pépins de courge, l'huile de germes de blé, l'huile de jojoba, l'huile de lin, l'huile de macadamia, l'huile de germes de maïs, l'huile de noisette, l'huile de noix, l'huile de vernonia, l'huile de noyaux d'abricot, l'huile d'olive, l'huile d'onagre, l'huile de palme, l'huile de passiflore, l'huile de pépins de raisin, l'huile de rosier, l'huile de ricin, l'huile de seigle, l'huile de sésame, l'huile de son de riz, l'huile de soja, l'huile de tournesol, et leurs mélanges, de préférence dans le groupe constitué par l'huile de noisette, l'huile de jojoba, l'huile de macadamia, l'huile de camélia, l'huile de coriandre, l'huile d'olive et leurs mélanges.

**17.** Composition selon l'une quelconque des revendications précédentes, dans laquelle la teneur totale en poids en huile(s) végétale(s) x est comprise entre 10% et 35% par rapport au poids total de la composition.

**18.** Composition selon l'une quelconque des revendications précédentes, dans laquelle la teneur totale en poids en monoalcool(s) en C2-C4 y est comprise entre 10% et 21% par rapport au poids total de la composition.

**19.** Procédé non thérapeutique de soin des matières kératiniques comprenant l'application sur les matières kératiniques d'une composition selon l'une quelconque des revendications précédentes.

**Patentansprüche**

**1.** Wasserfreie kosmetische Zusammensetzung, umfassend:

a) mindestens ein pflanzliches Öl mit einem Gesamtgewichtsgehalt x zwischen 10 % und 40 %, bezogen auf das Gesamtgewicht der Zusammensetzung; und
b) mindestens einen C2-C4-Monoalkohol mit einem Gesamtgewichtsgehalt y zwischen 5 % und 21 %, bezogen auf das Gesamtgewicht der Zusammensetzung; und
c) mindestens eine C-Verbindung, die sich von den das pflanzliche Öl bildenden Verbindungen unterscheidet, in einem Gesamtgewichtsgehalt z, bezogen auf das Gesamtgewicht der Zusammensetzung, wobei die C-Verbindung einen berechneten logP-Wert von kleiner oder gleich 8,6, vorzugsweise zwischen 4,0 und 8,6, bevorzugter zwischen 4,0 und 7,7, aufweist,

wobei:

- die C-Verbindung aus der Gruppe, die aus flüssigen Fettsäureestern, flüssigen Fettalkoholestern, flüssigen Fettalkoholen und deren Gemischen besteht, ausgewählt ist; und
- die Zusammensetzung keine Füllstoffe umfasst; und

$$90\% \leq x + y + z \leq 100\,\%;$$

und

$$z \geq (0{,}21y - 0{,}56)\,x.$$

**2.** Zusammensetzung nach Anspruch 1, wobei $95\,\% \leq x + y + z \leq 100\,\%$, vorzugsweise $97\% \leq x + y + z \leq 100\,\%$.

**3.** Zusammensetzung nach Anspruch 1, wobei die Zusammensetzung keine Verbindungen umfasst, die aus der Gruppe ausgewählt sind, die aus die Etheroxiden, die lipophilen Geliermitteln und deren Gemischen besteht.

**4.** Zusammensetzung nach einem der vorstehenden Ansprüche, wobei der C2-C4-Monoalkohol aus der Gruppe, die aus Ethanol, Propanol, Isopropanol, Butan-1-ol, Isobutanol und deren Gemischen, vorzugsweise Ethanol, besteht, ausgewählt ist.

**5.** Zusammensetzung nach einem der vorstehenden Ansprüche wobei das pflanzliche Öl eine Dielektrizitätskonstante s bei 25°C von größer oder gleich 2,5 aufweist.

**6.** Zusammensetzung nach einem der vorstehenden Ansprüche, wobei die Zusammensetzung keine von C-Verbindung verschiedenen flüssigen Fettsäureester oder flüssigen Fettalkoholester oder andere flüssige Fettsäureester als diejenigen, die das pflanzliche Öl bilden, umfasst.

**7.** Zusammensetzung nach einem der vorstehenden Ansprüche, wobei die C-Verbindung pflanzlichen Ursprungs ist.

**8.** Zusammensetzung nach einem der vorstehenden Ansprüche, wobei die C-Verbindung aus der Gruppe, die aus die flüssigen Fettsäuremonoestern, die flüssigen Fettalkoholmonoestern und deren Gemischen besteht, ausgewählt ist.

**9.** Zusammensetzung nach Anspruch 8, wobei die flüssigen Fettsäuremonoester der Formel $R_1C(=O)OR_1{}'$ entsprechen, wobei:

- $R_1$ einen linearen oder verzweigten Alkylrest darstellt, der 3 bis 15 Kohlenstoffatome, vorzugsweise 3 bis 9 Kohlenstoffatome, bevorzugter 5 bis 9 Kohlenstoffatome umfasst; und
- $R_1{}'$ einen linearen oder verzweigten Alkylrest darstellt, der 3 bis 10 Kohlenstoffatome umfasst.

**10.** Zusammensetzung nach Anspruch 9, wobei die Gesamtzahl der Kohlenstoffatome, die in den Resten $R_1$ und $R_1{}'$ enthalten sind, zwischen 8 und 22, vorzugsweise zwischen 9 und 18, beträgt.

**11.** Zusammensetzung nach einem der Ansprüche 1 bis 7, wobei die C-Verbindung aus der Gruppe, die aus die flüssigen Fettsäurediestern, die flüssigen Fettalkoholdiestern, die flüssigen Fettsäuretriestern, die flüssigen Fettalkoholtriestern und deren Gemischen besteht, ausgewählt ist.

**12.** Zusammensetzung nach einem der Ansprüche 1 bis 7, wobei die C-Verbindung aus der Gruppe, die aus den Verbindungen mit den folgenden INCI-Bezeichnungen besteht, ausgewählt ist: Isononylisononanoat, Dibutyladipat, Propylheptylcaprylat, Dicaprylylcarbonat, Caprylylcaprylat, Caprylylcaprat, Isopropylpalmitat, Diisopropylsebacat, Isodecylneopentanoat, Hexyllaurat, Isopropylmyristat, Isopropylpalmitat, Ethylhexylcaprylat, Ethylhexylcaprat, Triheptanoin und deren Gemischen.

**13.** Zusammensetzung nach einem der Ansprüche 1 bis 7, wobei die C-Verbindung unter die ungesättigten linearen flüssigen Fettalkoholen, vorzugsweise unter die ungesättigten linearen flüssigen Fettalkoholen mit 11 bis 18 Kohlenstoffatomen, ausgewählt ist.

**14.** Zusammensetzung nach einem der Ansprüche 1 bis 7, wobei die C-Verbindung unter die verzweigten flüssigen Fettalkoholen, vorzugsweise unter die verzweigten flüssigen Fettalkoholen mit 12 bis 24 Kohlenstoffatomen, ausgewählt ist.

**15.** Zusammensetzung nach einem der Ansprüche 1 bis 7, wobei die C-Verbindung aus die flüssigen Fettalkoholen ausgewählt ist, die aus der Gruppe ausgewählt sind, die aus Linolalkohol, Linolenalkohol, Ölalkohol, Isocetylalkohol, Isostearylalkohol, Undecylenalkohol, 2-Octyl-1-dodecanol, 2-Butyl-1-octanol, 2-Hexyl-1-decanol, 2-Octyl-1-dodecanol und deren Gemischen, vorzugsweise 2-Octyl-1- dodecanol, besteht.

**16.** Zusammensetzung nach einem der vorstehenden Ansprüche, wobei das pflanzliche Öl aus der Gruppe ausgewählt ist, die aus Leindotteröl, Süßmandelöl, Arganöl, Avocadoöl, Erdnussöl, Kamelienöl, Distelöl, Calophyllumöl, Rapsöl, Kopraöl, Korianderöl, Baumwollsamenöl, Kürbiskernöl, Weizenkeimöl, Jojobaöl, Leinöl, Macadamiaöl, Maiskeimöl, Haselnussöl, Walnussöl, Vernoniaöl, Aprikosenkernöl, Olivenöl, Nachtkerzenöl, Palmöl, Passionsblumenöl, Traubenkernöl, Rosenöl, Rizinusöl, Roggenöl, Sesamöl, Reiskleieöl, Sojaöl, Sonnenblumenöl und deren Gemischen besteht, vorzugsweise aus der Gruppe die aus Haselnussöl, Jojobaöl, Macadamiaöl, Kamelienöl, Korianderöl, Olivenöl und deren Gemischen, besteht.

**17.** Zusammensetzung nach einem der vorstehenden Ansprüche, wobei der Gesamtgewichtsgehalt an dem/den Pflanzenöl(en) **x** zwischen 10 % und 35 %, bezogen auf das Gesamtgewicht der Zusammensetzung, beträgt.

**18.** Zusammensetzung nach einem der vorstehenden Ansprüche, wobei der Gesamtgewichtsgehalt an C2-C4-Monoalkohol(en) **y** zwischen 10 % und 21 %, bezogen auf das Gesamtgewicht der Zusammensetzung, beträgt.

**19.** Nicht-therapeutisches Verfahren zur Pflege von Keratinmaterialien, umfassend das Auftragen einer Zusammensetzung nach einem der vorstehenden Ansprüche auf die Keratinmaterialien.

**Claims**

**1.** An anhydrous cosmetic composition comprising:

a) at least one vegetable oil in a total content in weight **x** of between 10% and 40% relative to the total weight of the composition; and
b) at least one C2-C4 monoalcohol, in a total content by weight **y** of between 5% and 21% relative to the total weight of the composition; and
c) at least one compound C different from the compounds constituting the vegetable oil, in a total content by weight **z** relative to the total weight of the composition, the compound C having a calculated logP value less than or equal to 8.6, preferably between 4.0 and 8.6, more preferably between 4.0 and 7.7,

in which:

- the compound C is selected from the group consisting of the liquid fatty acid esters, the liquid fatty alcohol esters, the liquid fatty alcohols and mixtures thereof; and
- the composition comprises no fillers; and

$$90\% \leq x + y + z \leq 100\%;$$

and

$$z \geq (0.21y - 0.56)\ x.$$

2. The composition according to claim 1, wherein $95\% \leq x + y + z \leq 100\%$, preferably $97\% \leq x + y + z \leq 100\%$.

3. The composition of claim 1, wherein the composition does not comprise compounds selected from the group consisting of ether oxides, the lipophilic gelling agents and mixtures thereof.

4. The composition according to any one of the preceding claims, wherein the C2-C4 monoalcohol is chosen from the group consisting of ethanol, propanol, isopropanol, butan-1-ol, isobutanol and mixtures thereof, preferably ethanol.

5. The composition according to any one of the preceding claims, wherein the vegetable oil has a dielectric constant $\varepsilon$ at 25°C greater than or equal to 2.5.

6. The composition according to any one of the preceding claims, wherein the composition does not comprise liquid fatty acid esters or liquid fatty alcohol esters other than the compound C or liquid fatty acid esters other than those constituting vegetable oil.

7. The composition according to any one of the preceding claims, wherein the compound C is of plant origin.

8. The composition according to any one of the preceding claims, wherein the compound C is chosen from the group consisting of liquid fatty acid monoesters, liquid fatty alcohol monoesters and mixtures thereof.

9. The composition according to claim 8, wherein the liquid fatty acid monoesters correspond to the formula $R_1C(=O)OR_1'$, wherein :

- $R_1$ represents a linear or branched alkyl radical comprising from 3 to 15 carbon atoms, preferably from 3 to 9 carbon atoms, more preferably from 5 to 9 carbon atoms; and
- $R_1'$ represents a linear or branched alkyl radical containing from 3 to 10 carbon atoms.

10. The composition according to claim 9, wherein the total number of carbon atoms in the radicals $R_1$ and $R_1'$ is between 8 and 22, preferably between 9 and 18.

11. The composition according to any one of claims 1 to 7, wherein the compound C is selected from the group consisting of the liquid fatty acid diesters, the liquid fatty alcohol diesters, the liquid fatty acid triesters, the liquid fatty alcohol triesters and mixtures thereof.

12. The composition according to any one of claims 1 to 7, wherein the compound C is chosen from the group consisting of compounds with the following INCI name: Isononyl Isononanoate, Dibutyl Adipate, Propylheptyl Caprylate, Dicaprylyl Carbonate, Caprylyl Caprylate, Caprylyl Caprate, Isopropyl Palmitate, Diisopropyl Sebacate, Isodecyl Neopentanoate, Hexyl Laurate, Isopropyl Myristate, Isopropyl Palmitate, Ethylhexyl Caprylate, Ethylhexyl Caprate, Triheptanoin and mixtures thereof.

13. The composition according to any one of claims 1 to 7, wherein the compound C is chosen from the linear unsaturated liquid fatty alcohols, preferably from linear unsaturated liquid fatty alcohols comprising from 11 to 18 carbon atoms.

14. The composition according to any one of claims 1 to 7, wherein the compound C is chosen from the branched liquid fatty alcohols, preferably from the branched liquid fatty alcohols comprising from 12 to 24 carbon atoms.

15. The composition according to any one of claims 1 to 7, wherein the compound C is chosen from liquid fatty alcohols selected from the group consisting of linoleic alcohol, linolenic alcohol, oleic alcohol, isocetyl alcohol, isostearyl alcohol, undecylenic alcohol, 2-octyl-1-dodecanol, 2-butyl-1-octanol, 2-hexyl-1-decanol, 2-octyl-1-dodecanol and mixtures thereof, preferably 2-octyl-1-dodecanol.

16. The composition according to any one of the preceding claims, wherein the vegetable oil is selected from the group consisting of camelina oil, sweet almond oil, argan oil, avocado oil, peanut oil, camellia oil, safflower oil, calophyllum oil, rapeseed oil, coconut oil, coriander oil, cottonseed oil, pumpkin seed oil, wheat germ oil, jojoba oil, linseed oil, macadamia oil, corn germ oil, hazelnut oil, walnut oil, vernonia oil, apricot kernel oil, olive oil, evening primrose oil, palm oil, passionflower oil, grapeseed oil, rosehip oil, castor oil, rye oil, sesame oil, rice bran oil, soya oil, sunflower oil, and mixtures thereof, preferably from the group consisting of hazelnut oil, jojoba oil, macadamia oil, camellia oil, coriander oil, olive oil and mixtures thereof.

17. The composition according to any one of the preceding claims, wherein the total content by weight of vegetable oil(s) x is between 10% and 35% relative to the total weight of the composition.

18. The composition according to any one of the preceding claims, wherein the total content by weight of C2-C4 y monoalcohol(s) is between 10% and 21% relative to the total weight of the composition.

19. A non-therapeutic method for caring for keratin materials comprising the application to the keratin materials of a composition according to any one of the preceding claims.

**EP 3 833 447 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1271011